# EUROPÄISCHE PATENTSCHRIFT

(11) **EP 3 203 979 B1**
(45) Veröffentlichungstag und Bekanntmachung des Hinweises auf die Patenterteilung: **29.01.2020**
(21) Anmeldenummer: 15767519.0
(22) Anmeldetag: 25.09.2015
(51) Int. Cl.: A61K 8/44, A61K 8/04, A61K 8/06, B65D 83/38, B65D 83/62

(54) **EMULSIONSSPRAY**
EMULSION SPRAY
SPRAY D'EMULSION

(30) Priorität: 09.10.2014 DE 102014220456
(43) Veröffentlichungstag der Anmeldung: 16.08.2017
(73) Patentinhaber: Beiersdorf AG, 20253 Hamburg (DE)
(72) Erfinder: SKUBSCH, Kerstin, 25497 Prisdorf (DE); LÜTTIG, Kaja, 20253 Hamburg (DE); MÜLLER, Claudia, 25499 Tangstedt (DE); WERNER, Regine, 29553 Bienenbüttel (DE)
(86) Internationale Anmeldenummer: PCT/EP2015/072077
(87) Internationale Veröffentlichungsnummer: WO 2016/055285

(56) Entgegenhaltungen:
- EP-A2- 2 783 676
- WO-A1-2014/105878
- WO-A2-2010/138266
- DE-A1-102008 021 631
- DE-A1-102010 050 774
- US-A1- 2014 186 411
- Anonymous: "GNPD - Moisturising Sun Lotion SPF 50+", , 1. August 2014 (2014-08-01), XP055221759, Gefunden im Internet: URL:http://www.gnpd.com/sinatra/recordpage /2624935/from_search/ZcQBsFUZvR/ [gefunden am 2015-10-19]
- Anonymous: "GNPD - Eczema Relief Spray", , 1. August 2014 (2014-08-01), XP055221755, Gefunden im Internet: URL:http://www.gnpd.com/sinatra/recordpage /2589939/from_search/ZcQBsFUZvR/ [gefunden am 2015-10-19]
- Anonymous: "Technology| BoV | Bag on Valve system | Aerosols filling | Pump Spray | Bag On Valve", , 9. September 2013 (2013-09-09), XP055221968, Gefunden im Internet: URL:https://web.archive.org/web/2013090901 3727/http://www.bagonvalve.com/technology/ [gefunden am 2015-10-19]

## Beschreibung

Die vorliegende Erfindung betrifft ein kosmetisches Spray bestehend aus einer Öl-in-Wasser-Emulsion (O/W-Emulsion) enthaltend Natriumstearoylglutamat sowie einem Sprühapplikator-System.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren haben dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Insbesondere wenn es sich um Emulsionen handelt, die mit einem Sprühapplikator (z.B. einer Aerosoldose oder einem Bag-on-Valve-System, siehe unten) aus einem Vorratsbehältnis unter Druck direkt auf die Haut aufgetragen werden, tritt das Problem auf, dass die Zubereitungen einerseits Temperatur- und lagerstabil sein sollen und nicht zur vorzeitigen Phasentrennung neigen und andererseits hinreichend dünnflüssig sein müssen, um überhaupt versprühbar zu sein.

Ferner tritt bei diesen durch Überdruck freigesetzten Zubereitungen immer das Problem auf, dass der mit Hilfe eines Dispensers erzeugte Sprühnebel zwei scheinbar widersprüchliche Eigenschaften aufweisen soll: Einerseits sollen die Sprühnebel-Tröpfchen möglichst klein und fein verteilt vorliegen, andererseits soll der Sprühnebel insgesamt relativ gut fokussiert aus dem System austreten um eine gezielte Anwendung auf definierte Hautpartien zu ermöglichen. Diese Eigenschaften werden natürlich zum einen durch die Ausgestaltung des Sprühkopfes und des Dispensers beeinflusst. Andererseits haben aber auch die Zusammensetzung und deren rheologischen Eigenschaften einen Einfluss auf die Tröpfchengröße und das "Ausbringungsverhalten" des Sprühnebels. Herkömmliche Zubereitungen des Standes der Technik weisen dabei den Nachteil auf, dass die Zubereitungen entweder zu Strahlartig ("wie aus einer Wasserpistole geschossen") aus dem Applikator-System austreten oder der Sprühnebel so fein verteilt bzw. in seiner Tröpfchengröße so heterogen wird, dass nur ein Teil der Zubereitung das vorgesehene Ziel erreicht, während ein relativ großer Teil der Tröpfchen eine abweichende Richtung nimmt und u.a. vorzeitig zu Boden sinkt. Dies führt bei der Anwendung des Kosmetikums dann zu einer ungewünschten Verunreinigung der Anwendungsumgebung mit der Zubereitung.

Es war daher die Aufgabe der vorliegenden Erfindung, die Mängel des Standes der Technik zu beseitigen und ein kosmetisches Spray zu entwickeln, dessen Sprühbild einen fokussierten feinen Sprühnebel ermöglicht, ohne dass es zu größeren "Streuverlusten" kommt. Darüber hinaus sollte die versprühte Zubereitung beim Auftreffen auf der Haut gut auf dieser anhaften und nicht wieder von ihr Abprallen.

Überraschend gelöst wird die Aufgabe durch eine kosmetisches Spray gemäß Anspruch 1.

Durch den Überdruck im Druckgasbehälter wird bei diesen Bag-on-Valve-Systemen beim Öffnen des Sprühkopfes der Inhalt des Beutels (hier also die O/W-Emulsion) durch den Sprühkopf nach außen gedrückt und durch den im Sprühkopf vorhandenen Dispenser in kleine Tröpfchen ("Sprühnebel") geteilt. Der Druckausgleich findet also nicht durch das direkte Entweichen des Druckgases aus dem Überdruckgefäß statt, sondern durch das Entleeren des Inhaltes aus dem Vorratsbeutel.

Es ist erfindungsgemäß vorteilhaft, wenn der Sprühkopf des Sprühapplikators einen gleichmäßigen Sprühstrahl über den gesamten Lebenszeitraum der Dose aufweist. Aus einer Entfernung von 10 cm wird ein Sprühbild von 5 - 6 cm favorisiert. Beim Abfall des Druckes über den gesamten Lebenszeitraum der Dose sollte das Sprühbild nicht über 8 cm hinausgehen.

Erfindungsgemäß besonders bevorzugt ist ein Sprühapplikator mit der folgenden Spezifikation: 9 bar Überdruck mit Stickstoff
Ventil DU 2527 oder DU 3527 von Aptar®, BOV - Cup: Alu gold laquered - Inner gasket: Buna KA 6712 - Body valve: PP - Spring: Inox 302 - Piston: POM - External gasket: Butyl 1,2 mm Foil: PET12/ALU8/OPA15/PP75.

Erfindungsgemäß vorteilhafte Ausführungsformen der vorliegenden Erfindung sind dadurch gekennzeichnet, dass der Beutel, welcher die O/W-Emulsion enthält aus einem Laminat aus PE/adhesive/PA/adhesive/AL/adhesive/PET gebildet wird.

Zwar kennt der Stand der Technik die GNPD (Mintel) Datenbankeinträge Record ID 2624935 und 2589939 sowie die US 2014/0186411 A1, WO 2010/138266 A2, DE 102010050774 A1, WO 2014/105878 A1, EP 2783676 A2, DE 102008021631 A1 und "Technoloy/BoV/Bag on Valve System/aerosols filling/Pump Sptray/Bag On Valve", doch konnten diese Schriften nicht den Weg zur vorliegenden Erfindung weisen.

Es ist vorteilhaft im Sinne der vorliegenden Erfindung, wenn die erfindungsgemäße O/W-Emulsion von 0,1 bis 0,5 Gewichts-% Natriumstearoylglutamat, bezogen auf das Gesamtgewicht der Emulsion enthält.

Es ist bevorzugt im Sinne der vorliegenden Erfindung, wenn die erfindungsgemäße O/W-Emulsion von 0,2 bis 0,3 Gewichts-% Natriumstearoylglutamat, bezogen auf das Gesamtgewicht der Emulsion enthält.

Es ist erfindungsgemäß vorteilhaft, wenn die erfindungsgemäße O/W-Emulsion Cetearylalkohol enthält.

Enthält die O/W-Emulsion Cetearylalkohol, so ist es erfindungsgemäß vorteilhaft, wenn dieser Stoff in einer Konzentration von 0,5 bis 3 Gewichts-%, bezogen auf das Gesamtgewicht der Emulsion in dieser vorhanden ist.

Es ist erfindungsgemäß vorteilhaft, wenn die Emulsion Dicaprylylether, Isopropylpalmitat und/oder Sheabutter enthält.

Enthält die Emulsion Dicaprylylether, so ist es erfindungsgemäß vorteilhaft, wenn dieser Stoff in einer Konzentration von 1 bis 7 Gewichts-%, bezogen auf das Gesamtgewicht der Emulsion in dieser vorhanden ist.

Enthält die Emulsion Isopropylpalmitat, so ist es erfindungsgemäß vorteilhaft, wenn dieser Stoff in einer Konzentration von 1 bis 7Gewichts-%, bezogen auf das Gesamtgewicht der Emulsion in dieser vorhanden ist.

Enthält die Emulsion Sheabutter, so ist es erfindungsgemäß vorteilhaft, wenn dieser Stoff in einer Konzentration von 0,5 bis 3 Gewichts-%, bezogen auf das Gesamtgewicht der Emulsion in dieser vorhanden ist.

Erfindungsgemäß vorteilhafte Ausführungsformen der vorliegenden Erfindung sind auch dadurch gekennzeichnet, dass die Emulsion Dimethicone und/oder Cyclomethicon enthält.

Die Ölphase der erfindungsgemäßen Emulsion kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin, C15-19 Alkane und Isohexadecan. Unter den Polyolefinen sind Polydecene die bevorzugten Substanzen.

Es ist erfindungsgemäß von Vorteil, wenn die erfindungsgemäße Emulsion Ethanol und/oder Glycerin enthält.

Enthält die Emulsion Ethanol, so ist eine Einsatzkonzentration von 0,5 bis 8 Gewichts-%, bezogen auf das Gesamtgewicht der Emulsion erfindungsgemäß vorteilhaft.

Enthält die Emulsion Glycerin, so ist eine Einsatzkonzentration von 1 bis 12 Gewichts-%, bezogen auf das Gesamtgewicht der Emulsion erfindungsgemäß vorteilhaft.

Vorteilhafte Ausführungsformen der vorliegenden Erfindung sind auch dadurch gekennzeichnet, dass die Emulsion einen oder mehrere Wirkstoffe gewählt aus der Gruppe der Verbindungen UV-Filter, Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Erfindungsgemäß vorteilhafte UV-Filter können beispielsweise, gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhe-xylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß von Vorteil, wenn die Emulsion Ethylhexylglycerin, Propylenglycol, Butylenglycol, 2-Methylpropan-1,3-diol, 1,2-Pentandiol, 1,2-Hexandiol, 1,2-Octandiol, Piroctone Olamin und/oder 1,2-Decandiol enthält.

Erfindungsgemäß vorteilhafte Ausführungsformen der vorliegenden Erfindung sind dadurch gekennzeichnet, dass die Emulsion Phenoxyethanol und/oder Methylparaben enthält. Dabei ist es erfindungsgemäß vorteilhaft, wenn die Emulsion frei ist von Propyl- und Butylparaben.

Erfindungsgemäß vorteilhafte Ausführungsformen sind ferner dadurch gekennzeichnet, dass die Emulsion mindestens 70 Gewichts-% Wasser, bezogen auf das Gesamtgewicht der Emulsion, enthält.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | A | B | C | D |
|---|---|---|---|---|
| | % | % | % | % |
| Natriumstearoyl Glutamat | 0,2 | 0,2 | 0,3 | 0,25 |
| Xanthan Gummi | | 0,1 | | |
| Carbomer | 0,2 | 0,1 | | |
| Caprylic/Capric Triglycerid | | | 3 | |
| Isopropyl Palmitat | 3,5 | 3,5 | | |
| Shea Butter | 1 | | 1 | |
| Kakaobutter | | 1 | | |
| Dimethicon | 0,9 | 1 | | |
| Mandelöl | | | 1 | |
| Cetearyl Alkohol | 1 | 1 | 1,5 | |
| Dicaprylyl Ether | 2 | | 3 | |
| Glycerin | 7 | 5 | 10 | 8 |

| Natronlauge Lösung | pH Wert Einstellung | pH Wert Einstellung | pH Wert Einstellung | pH Wert Einstellung |
|---|---|---|---|---|
| Phenoxyethanol | 0,5 | 0,5 | 0,5 | 0,5 |
| Methylparaben | 0,3 | 0,2 | 0,3 | 0,3 |
| Alkohol | 3 | | 2 | 1 |
| Wasser | ad. 100 | ad. 100 | ad. 100 | ad. 100 |
| Anfangsdruck in bar | 7 | 8 | 7 | 8 |
| Druckgas | Stickstoff | Stickstoff | Stickstoff | Stickstoff |
| Sprühventil | DU 3527 | DU 3527 | DU 3527 | DU 3527 |

| | | | | |
|---|---|---|---|---|
| BOV Applikator System z.B. von der Firma Aptar® Ex-EP BOV - Cup: Alu gold laquered - Inner gasket: Buna KA 6712 - Body valve: PP - Spring: Inox 302 - Piston: POM - External gasket: Butyl 1,2 mm Foil: PET12/ALU8/OPA15/PP75 | | | | |

## Patentansprüche

1. Kosmetisches Spray bestehend aus
a) einer Öl-in-Wasser-Emulsion (O/W-Emulsion) enthaltend Natriumstearoylglutamat und Ethanol sowie
b) einem Sprühapplikator-System, wobei als Sprühapplikator-System ein Bag-on-valve Applikator System eingesetzt wird, bei dem sich in einem Druckgasbehälter mit Überdruck ein Beutel enthaltend die O/W-Emulsion befindet, **dadurch gekennzeichnet, dass** der Überdruck in dem Überdruckbehälter des Bag-on-valve-Applikators von 2 bis 12 bar beträgt.

2. Kosmetisches Spray nach Anspruch 1, **dadurch gekennzeichnet, dass** der Sprühapplikator die folgenden Spezifikation aufweist: 9 bar Überdruck mit Stickstoff Ventil DU 2527 oder DU 3527 von Aptar®, BOV - Cup: Alu gold laquered - Inner gasket: Buna KA 6712 - Body valve: PP - Spring: Inox 302 - Piston: POM External gasket: Butyl 1,2 mm Foil: PET12/ALU8/OPA15/PP75.

3. Kosmetisches Spray nach einem der vorhergehenden Ansprüche, **dadurch gekennzeichnet, dass** der Beutel, welcher die O/W-Emulsion enthält aus einem Laminat aus PE/adhesive/PA/adhesive/AL/adhesive/PET gebildet wird.

4. Kosmetisches Spray nach einem der vorhergehenden Ansprüche, **dadurch gekennzeichnet, dass** die O/W-Emulsion von 0,1 bis 0,5 bis Gewichts-% Natriumstearoylglutamat, bezogen auf das Gesamtgewicht der Emulsion enthält.

5. Kosmetisches Spray nach einem der vorhergehenden Ansprüche, **dadurch gekennzeichnet, dass** die O/W-Emulsion Cetearylalkohol enthält.

6. Kosmetisches Spray nach einem der vorhergehenden Ansprüche, **dadurch gekennzeichnet, dass** die O/W-Emulsion von 0,5 bis 3 bis Gewichts-% Cetearylalkohol, bezogen auf das Gesamtgewicht der Emulsion enthält.

7. Kosmetisches Spray nach einem der vorhergehenden Ansprüche, **dadurch gekennzeichnet, dass** die Emulsion Dicaprylylether, Isopropylpalmitat und/oder Sheabutter enthält.

8. Kosmetisches Spray nach einem der vorhergehenden Ansprüche, **dadurch gekennzeichnet, dass** die Emulsion Dimethicone und/oder Cyclomethicon enthält.

9. Kosmetisches Spray nach einem der vorhergehenden Ansprüche, **dadurch gekennzeichnet, dass** die Emulsion Glycerin enthält.

10. Kosmetisches Spray nach einem der vorhergehenden Ansprüche, **dadurch gekennzeichnet, dass** die Emulsion einen oder mehrere Wirkstoffe gewählt aus der Gruppe der Verbindungen UV-Filter, Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

11. Kosmetisches Spray nach einem der vorhergehenden Ansprüche, **dadurch gekennzeichnet, dass** die Emulsion Propylenglycol, Butylenglycol, 2-Methylpropan-1,3-diol, 1,2-Pentandiol, 1,2-Hexandiol, 1,2-Octandiol und/oder 1,2-Decandiol enthält.

12. Kosmetisches Spray nach einem der vorhergehenden Ansprüche, **dadurch gekennzeichnet, dass** die Emulsion Phenoxyethanol und/oder Methylparaben enthält.

13. Kosmetisches Spray nach einem der vorhergehenden Ansprüche, **dadurch gekennzeichnet, dass** die Emulsion mindestens 70 Gewichts-% Wasser, bezogen auf das Gesamtgewicht der Emulsion, enthält.

## Claims

1. Cosmetic spray consisting of
a) an oil-in-water emulsion (O/W emulsion) comprising sodium stearoyl glutamate and ethanol and also
b) a spray applicator system, wherein the spray applicator system used is a bag-on-valve applicator system, in which a bag containing the O/W emulsion is in a pressurized gas container under positive pressure, **characterized in that** the positive pressure in the positive pressure container of the bag-on-valve applicator is from 2 to 12 bar.

2. Cosmetic spray according to Claim 1, **characterized in that** the spray applicator has the following specification: 9 bar positive pressure with nitrogen valve DU 2527 or DU 3527 from Aptar®, BOV - cup: Alu gold lacquered - inner gasket: Buna KA 6712 - body valve: PP - spring: Inox 302 - piston: POM external gasket: butyl 1.2 mm foil: PET12/ALU8/OPA15/PP75.

3. Cosmetic spray according to either of the preceding claims, **characterized in that** the bag containing the O/W emulsion is formed from a laminate of PE/adhesive/PA/adhesive/AL/adhesive/PET.

4. Cosmetic spray according to any of the preceding claims, **characterized in that** the O/W emulsion comprises from 0.1 to 0.5 to % by weight sodium stearoyl glutamate, based on the total weight of the emulsion.

5. Cosmetic spray according to any of the preceding claims, **characterized in that** the O/W emulsion comprises cetearyl alcohol.

6. Cosmetic spray according to any of the preceding claims, **characterized in that** the O/W emulsion comprises from 0.5 to 3 to % by weight cetearyl alcohol, based on the total weight of the emulsion.

7. Cosmetic spray according to any of the preceding claims, **characterized in that** the emulsion comprises dicaprylyl ether, isopropyl palmitate and/or shea butter.

8. Cosmetic spray according to any of the preceding claims, **characterized in that** the emulsion comprises dimethicone and/or cyclomethicone.

9. Cosmetic spray according to any of the preceding claims, **characterized in that** the emulsion comprises glycerol.

10. Cosmetic spray according to any of the preceding claims, **characterized in that** the emulsion comprises one or more active ingredients selected from the group of compounds comprising UV filters, magnolia extract, glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

11. Cosmetic spray according to any of the preceding claims, **characterized in that** the emulsion comprises propylene glycol, butylene glycol, 2-methylpropane-1,3-diol, 1,2-pentanediol, 1,2-hexanediol, 1,2-octanediol and/or 1,2-decanediol.

12. Cosmetic spray according to any of the preceding claims, **characterized in that** the emulsion comprises phenoxyethanol and/or methylparaben.

13. Cosmetic spray according to any of the preceding claims, **characterized in that** the emulsion comprises at least 70% by weight water, based on the total weight of the emulsion.

## Revendications

1. Spray cosmétique constitué par
a) une émulsion huile-dans-eau (émulsion H/E) contenant du stéaroylglutamate de sodium et de l'éthanol ainsi que par
b) un système applicateur par pulvérisation, un système applicateur à valve à sachet ("bag-on-valve") étant utilisé comme système applicateur par pulvérisation, dans lequel un sachet contenant l'émulsion H/E se trouve dans un récipient à gaz sous pression sous une surpression, **caractérisé en ce que** la surpression dans le récipient à surpression de l'applicateur à valve à sachet est de 2 à 12 bars.

2. Spray cosmétique selon la revendication 1, **caractérisé en ce que** l'applicateur par pulvérisation présente la spécification suivante : surpression de 9 bars avec une soupape à azote DU 2527 ou DU 3527 d'Aptar®, embout à soupape de surpression ("BOV - Cup") : aluminium laqué doré - joint interne : Buna KA 6712 - soupape de corps : PP - ressort : Inox 302 - piston : POM - joint externe : butyl 1,2 mm - Feuille : PET12/ALU8/OPA15/PP75.

3. Spray cosmétique selon l'une quelconque des revendications précédentes, **caractérisé en ce que** le sachet, qui contient l'émulsion H/E, est formé à partir d'un stratifié en PE/adhésif/PA/adhésif/AI/adhésif/PET.

4. Spray cosmétique selon l'une quelconque des revendications précédentes, **caractérisé en ce que** l'émulsion H/E contient 0,1 à 0,5 % à en poids de stéaroylglutamate de sodium, par rapport au poids total de l'émulsion.

5. Spray cosmétique selon l'une quelconque des revendications précédentes, **caractérisé en ce que** l'émulsion H/E contient de l'alcool cétéarylique.

6. Spray cosmétique selon l'une quelconque des revendications précédentes, **caractérisé en ce que** l'émulsion H/E contient 0,5 à 3 % à en poids d'alcool cétéarylique, par rapport au poids total de l'émulsion.

7. Spray cosmétique selon l'une quelconque des revendications précédentes, **caractérisé en ce que** l'émulsion contient du dicaprylyléther, du palmitate d'isopropyle et/ou du beurre de karité.

8. Spray cosmétique selon l'une quelconque des revendications précédentes, **caractérisé en ce que** l'émulsion contient du diméthicone et/ou du cyclométhicone.

9. Spray cosmétique selon l'une quelconque des revendications précédentes, **caractérisé en ce que** l'émulsion contient du glycérol.

10. Spray cosmétique selon l'une quelconque des revendications précédentes, **caractérisé en ce que** l'émulsion contient une ou plusieurs substances actives choisies dans le groupe des composés de type filtre UV, extrait de magnolia, acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

11. Spray cosmétique selon l'une quelconque des revendications précédentes, **caractérisé en ce que** l'émulsion contient du propylèneglycol, du butylèneglycol, du 2-méthylpropane-1,3-diol, du 1,2-pentanediol, du 1,2-hexanediol, du 1,2-octanediol et/ou du 1,2-décanediol.

12. Spray cosmétique selon l'une quelconque des revendications précédentes, **caractérisé en ce que** l'émulsion contient du phénoxyéthanol et/ou du méthylparabène.

13. Spray cosmétique selon l'une quelconque des revendications précédentes, **caractérisé en ce que** l'émulsion contient au moins 70 % en poids d'eau, par rapport au poids total de l'émulsion.
